# EUROPEAN PATENT APPLICATION

(11) **EP 2 119 465 A1**
(43) Date of publication of application: **18.11.2009**
(21) Application number: 08156374.4
(22) Date of filing: 16.05.2008
(51) Int. Cl.: A61M 15/00, B05B 17/06, A61M 16/00

(54) **Nebulizer with breathing phase detecting sensor for delivering nebulized drugs to a user**

(71) Applicant: Markos Mefar S.P.A., 25073 Bovezzo (IT)
(72) Inventor: Rivetti, Alberto, 25073 Bovezzo (IT); Giaretta, Mariano, Liscate, MI (IT)
(74) Representative: Pittis, Olivier

(57) **Abstract**

Nebulizer and process for delivering a nebulized liquid solution, in particular an aerosolized drug. In the preferred embodiment, the nebulizer comprises a pump shaft (13) and further a vibrating element (5) for vibrating the pump shaft (13), and a mesh member (6) comprising one or several traversing holes (14) and cooperating with the pump shaft (13) for nebulizing the liquid. At least one sensor (7) is used for detecting at least an inspiration phase of the user. The vibrating element (5) vibrates the pump shaft (13) in response to the detection of an inspiration phase by said sensor (7) so as to deliver the nebulized liquid solution only during the inhalation phases of the user.

## Description

The present invention is directed to an electronic nebulizer for administering nebulized or aerosolized drugs, therapeutic agents or any other active products to the airways of a user, i.e. a human being.

It is well known to nebulize or aerosolize therapeutic agents, drugs or other active products, in particular when there are in the liquid form, in solution or suspension, in order to administer them to the airways of a user, such as a patient.

This can be done by means of a nebulizer or an atomizer using an electronic pump and a mesh member cooperating together for creating the aerolized drug-containing mist that can be inhaled by the user and subsequently delivered to the airways of the patient.

For instance, document US-A-4,796,807 discloses an ultrasonic atomizer for atomizing liquids, comprising a piezoelectric transducer mechanically coupled to an amplitude transformer having a free end on which an atomizing disk is mounted. A cap supports a sieve-like diaphragm, i.e. mesh member, and is mounted on the housing comprising the amplitude transformer so that the diaphragm rests on the atomizing disk. The sieve-like diaphragm is held elastically against the atomizing disk by means spring means.

Further, US-A-4,850,534 teaches a nebulizer which pumps up water and mists the pumped up water. The nebulizer comprises an elongated main body with a center hole for water passage, and piezoelectric vibration elements and electrodes for energizing the same. Upon vibration of the elements, water is pumped up through the inlet of the main body, and is dissipated into the air through the outlet of the main body. Preferably, the inlet and the outlet are removable from the main body, and the inlet is coated with a thin hard film. The outlet is covered with a mesh member.

Other similar devices or systems are disclosed by documents EP-A-933138 and EP-A-635312.

A problem that exists with these existing devices, is that the aerosol mist is generated as soon as the switch-on button of the device is actuated by the user and lasts as long as the switch-off button is not actuated again. This continuous nebulization during the use of the nebulizer may lead to loses of aerosolized product during the user's exhalation or during a delay between inhalation and exhalation. The amount of wasted aerosol may be difficult to quantify and some aerosol may be lost due to condensation on the user's interface, e.g. the mouthpiece, of the nebulizer during periods of "non-inhalation".

Further, if the user omits to switch-off the device, not only drug will be lost, but also energy as the device will continue to run and to consume battery power.

A goal of the present invention is then to provide an improved electronic nebulizer for delivering nebulized liquid compounds or active products to a user, in particular drugs or therapeutic agents, preferably having either a topical or a systemic action, or both, that overcomes at least some, but preferably most of or all, of the drawbacks and problems of the nebulizers of the prior art.

Another goal of the present invention is to provide an improved electronic nebulizer for delivering nebulized liquid compounds to the airways of the user, that is compact, noiseless, easily portable, patient friendly but preferably, at the same time, able to delivery constant and reproducible soft mist doses within a predetermined particle size range in order to deliver the nebulized liquid compounds to the desired portion of the lung, minimizing the losses of aerosolized product, allowing for a dose reduction and a clinical dose assurance.

Another goal of the present invention is to provide an improved electronic nebulizer able to provide these features synchronously, i.e. concurrently, with the start of the user's inhalation.

A solution according to the present invention is a nebulizer for delivering a nebulized liquid solution or suspension, such a liquid formulation containing one (or several) active pharmaceutical ingredients (APIs), medication, or similar, comprising a pump shaft and further comprising a vibrating element for vibrating the pump shaft, and a mesh member comprising one or several traversing holes and cooperating with the pump shaft for nebulizing the liquid, **characterized in that** it further comprises at least one sensor for detecting at least an inspiration phase of the user, and the vibrating element vibrates the pump shaft in response to the detection of an inspiration phase by said sensor.

In the spirit of the present invention :
- the terms "nebulizer" and "atomizer" are considered as totally equivalent for designating devices that are used for obtaining a mist, an aerosol or similar of one or several active products that is/are in liquid form, dissolved in a liquid, in suspension in a liquid or similar.
- the terms "mesh" or "sieve" are considered as totally equivalent for designating a thin plate comprising one or several holes, usually a plurality of small holes, and that is adapted to be used for generating a mist of liquid active product.
- the terms "active product", "drug", "pharmaceutical" compound or product, "medication", or similar are considered as being totally equivalent.
- the terms "liquid solution", "liquid suspension", "liquid formulation", "liquid drug" or similar are considered as being totally equivalent for designating one (or several) compound, drug, APIs, medication or similar that is in liquid form able to be aerosolized. Actually, the "active product(s)" or similar can be itself in liquid form, or it can be in solid form, such as in powder form, but dissolved, put in suspension, mixed or similar with a liquid and/or with appropriate vehicles or excipients.
- the terms "to nebulize", "to mist", "to aerosolize" and "to atomize" or similar are considered as totally equivalent.

The nebulizer of the present invention can comprise one or several of the following additional features :
- it further comprises a main body having a proximal portion forming a chamber comprising an outlet situated at an extremity of said proximal portion, and a vessel for receiving a liquid solution to be nebulized, and a pump device comprising the pump shaft.
- the pump shaft comprises an upper end, a lower end and a pump bore axially arranged through said pump shaft between the upper end and the lower end, said lower end being situated inside said vessel.
- the mesh member has a rear surface and a front surface, said rear surface facing the upper end of the pump shaft.
- the pump shaft being actuated by the vibrating element in such a way that the liquid solution pumped by the pump device in the vessel passes successively through the pump bore and through at least one hole from the rear surface in the direction of the front surface of the mesh member, and is subsequently atomized in the proximal portion and delivered by the outlet situated at an extremity of said proximal portion.
- it further comprises an electronic unit comprising a microprocessor and an interface circuit with comparator cooperating with sensor for determining the start and/or the end of inspiration and/or expiration phases, preferably the determination of the start and/or the end of an inspiration phase is based on a monitoring of the breath airflow of the user.
- the electronic unit further comprises an oscillator and vibrator driver adapted for controlling the vibrating element, especially for starting or for stopping a vibration of said vibrating element, preferably the oscillator and vibrator driver is adapted to deliver at least a high-frequency signal to the vibrating element. The most suitable high-frequency to be applied is easily determinable by empirical tests and it is in the range between 20 and 350 kHz.
- the oscillator and vibrator driver is controlled by the microprocessor, preferably via a gate.
- the sensor cooperates with an interface circuit with comparator for determining the start of an inspiration phase of the user, the end of an inspiration phase of the user, the start of an expiration phase of the user or the end of an expiration phase of the user, based on a monitoring of the breathing intensity of the user.
- the interface circuit with comparator cooperates with microprocessor for controlling the oscillator and vibrator driver so as to start the vibration of the vibrating element in response to the reception by the interface circuit with comparator of a signal coming from sensor indicating that the breathing intensity exceeds a given threshold, preferably said threshold corresponds to the start of an inspiration phase of the user.
- in one embodiment of the invention, the interface circuit with comparator is an interface circuit with hysteresis comparator and cooperates with microprocessor for controlling the oscillator and vibrator driver so as to stop the vibration of the vibrating element in response to the reception by the interface with hysteresis comparator of a signal coming from sensor indicating that the breathing intensity is below a given low threshold, preferably said low threshold corresponds to the end of an inspiration phase of the user. In other words, when, thanks to the sensor, the end of an inspiration phase or the start of an expiration phase of the user is detected, the oscillator and vibrator driver are deactivated, via gate, by the microprocessor and the vibration of the vibrating element is then stopped, preferably the sensor allows to detect the end of an inspiration phase, i.e. a low threshold level.
- in a second embodiment of the invention the interface circuit with comparator is an interface circuit with temporized comparator and cooperates with microprocessor for controlling the oscillator and vibrator driver so as to stop the vibration of the vibrating element after a preset duration.
- the sensor provides to the interface circuit with comparator, an analog signal proportional or related monotonically to the instantaneous breath intensity of the user.
- the vibrating element vibrates the pump shaft thereby atomizing the liquid solution pumped by the pump device only during the inspiration phases of the user, i.e. during the whole inspiration time or only during part of it.
- it further comprises a power-supply management unit cooperating with the microprocessor and with a switch that can be actuated by the user, so that the power-supply management unit is able to detect an actuation of the switch by the user and to send a signal to the microprocessor in response to such an actuation.
- the microprocessor, upon receiving of a signal from the power-supply management unit, is able to determine the duration of the actuation of the operation switch by the user and, when the microprocessor determines that the duration of the actuation of the switch by the user exceeds a given value, the nebulizer is operated in breath-actuated mode (BAM) and the sensor starts to detect the inspiration phases of the user.
- it further comprises battery means for electrically powering the power-supply management unit, the interface circuit with comparator, the microprocessor, one or several LEDs, the DC/DC converter and/or the oscillator and vibrator driver.
- the sensor is pneumatically linked to the proximal portion for sensing the gas pressure in said proximal portion, preferably by means of a hollow tube that transmits at least measurement to the sensor.
- said sensor is situated inside said proximal portion and electronically linked to said electronic unit.
- the electronic unit comprises an oscillator and vibrator driver connected to the battery unit so as to receive electric power from the battery unit, preferably the voltage delivered by the battery unit is converted by a voltage converter to a voltage adapted to the operation of the oscillator circuit, said voltage converter being arranged between the battery unit and the oscillator circuit.
- the interface circuit with hysteresis comparator internally splits a given threshold level into a high threshold and a lower threshold, respectively, for rising-edge and falling-edge transitions, in order to avoid chattering and ensure clean switching. For instance, the high threshold equals about 0,3 l/s and the low threshold equals about 0,1 l/s; the high threshold value is always higher than the low threshold value.
- an oscillator and vibrator driver connected to the battery unit so as to receive electric power from the battery unit, preferably the voltage delivered by the battery unit is converted by a voltage (DC/DC) converter to a voltage adapted to the operation of the oscillator and vibrator driver, said voltage converter being arranged between the battery unit and the oscillator and vibrator driver.
- the mesh member is a plate comprising a plurality of traversing holes.
- the pump device comprises at least one piezo-electric element arranged on the shaft, preferably several piezo-electric elements.
- the sensor is a flow sensor, preferably a thermoanemometric-type flow sensor.
- the proximal portion comprises at least one one-way exhalation valve for releasing the user's exhalation gas flow.
- it comprises mode-toggling means for toggling between the BAM mode and the CM mode, and vice versa, preferably the mode-toggling means comprise a press-button switch (29).

Further, the present invention also deals with a process for delivering a nebulized liquid solution by means of a nebulizer comprising a pump shaft, a vibrating element for vibrating the pump shaft, a vessel for receiving a liquid solution to be nebulized, and a mesh member comprising traversing holes and cooperating with the pump shaft for nebulizing the liquid, comprising the steps of:
a) using at least one sensor for detecting at least an inspiration phase of the user, and
b) vibrating the pump shaft by means of the vibrating element in response to the detection of an inspiration phase by said sensor thereby nebulizing at least part of the liquid solution.

Of course, the process of the present invention can also include one or several of the features listed above in connection with the nebulizer of the invention.

Several possible embodiments of a nebulizer according to the present invention are detailed hereafter and have been illustrated on the enclosed Figures, among which :
- Fig. 1a represents a schematic view of a nebulizer according to the present invention,
- Fig. 1b represents a schematic view of another embodiment of the top part of a nebulizer according to the present invention,
- Fig. 2 shows a pump device useable in the device of Fig. 1a or 1b,
- Fig. 3 shows a mesh member useable in the device of Fig. 1a or 1b, and
- Fig. 4 is a block diagram of the electronic unit used in the device of Fig. 1a or 1b,
- Fig. 5 and 6 are flow charts of two different embodiments of the way the electronic unit 10 of the nebulizer of the invention works, and
- Fig. 7 represents the way a nebulizer of the invention works during a breath cycle.

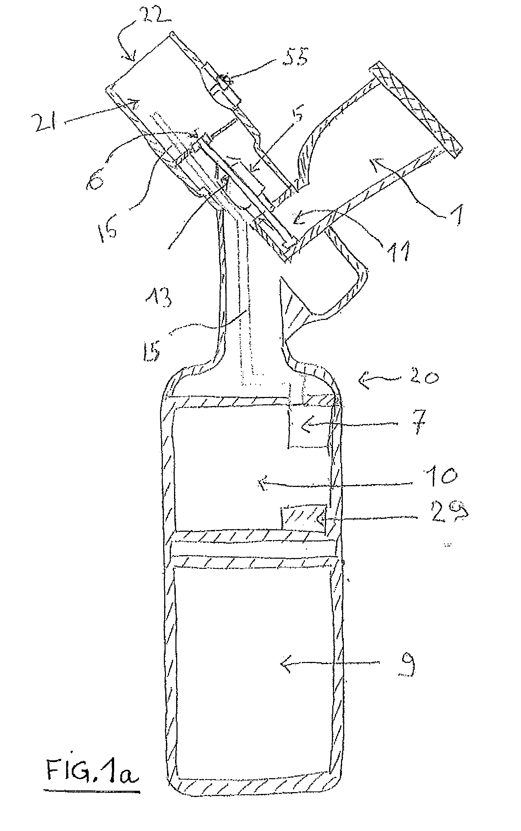

More precisely, Fig.1a represents a nebulizer according to the invention useable for distributing soft mists of an aerosolized or nebulized drug to the airways of a user. The nebulizer comprises a main body 20 having a proximal portion 21 forming a chamber with an outlet 22 situated at the extremity of said proximal portion 21 for delivering the atomized drug, the liquid to be atomized being stored in a liquid storage vessel 1.

The liquid storage vessel 1 can be a reservoir that may be refilled with some fresh liquid drug, once empty, or can be a refillable or non-refillable, disposable cartridge or container containing the liquid drug that is inserted inside a housing or a compartment that has a shape and form that are adapted to received such a cartridge.

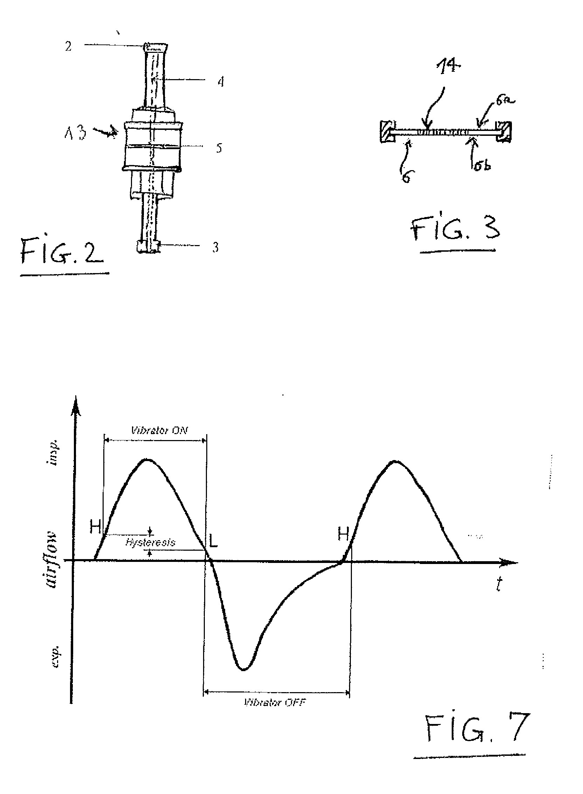

As shown on Fig. 2, a pump device 13, 2, 3, 4, 5 is used for pumping the liquid drug into the storage vessel 1. Said pump device is preferably an ultrasonic pump as such kind of pump allows to obtain a nebulizer that is compact and noiseless, and further offers a better control of the aerosolized particle sizes and their distribution.

Said pump comprises a pump shaft 13 having an upper end 2 and lower end 3, and a pump bore 4 is axially arranged through the pump shaft 13 between both ends 2, 3. In other words, the pump shaft 13 is hollow as a tube in which travels the liquid drug towards the mesh member 6.

A vibrator 5, preferably an ultrasonic vibrator, cooperates with the pump shaft 13 for vibrating said pump shaft 13, upwardly and downwardly, in such a way that the mesh member 6 works as a valve, opening and closing the end of the pump bore 4 situated at the upper end 2 of the pump shaft 13. Owing the valve action of the mesh member 6, the liquid pumped by the pump device 13, 2, 3, 4 in the liquid vessel 1 spreads over the upper end 2 of said pump shaft 13, when the pump bore 4 is open. When the pump bore 4 is closed, the pumping action is stopped and the liquid film is atomized through the holes 14 of the mesh member 6. The atomization occurs in the proximal portion 21 and subsequently delivered outside through the outlet 22 situated at the extremity of the proximal portion 21. Preferably, the pump device 13, 2, 3, 4,5 is an ultrasonic pump.

The vibrator 5 comprises one or several piezo-electric elements, preferably annular-shaped piezo-electric elements arranged on the peripheral external surface of the shaft 13. Such arrangements are well-known in the art and do not need to be further explained in the frame of the present invention.

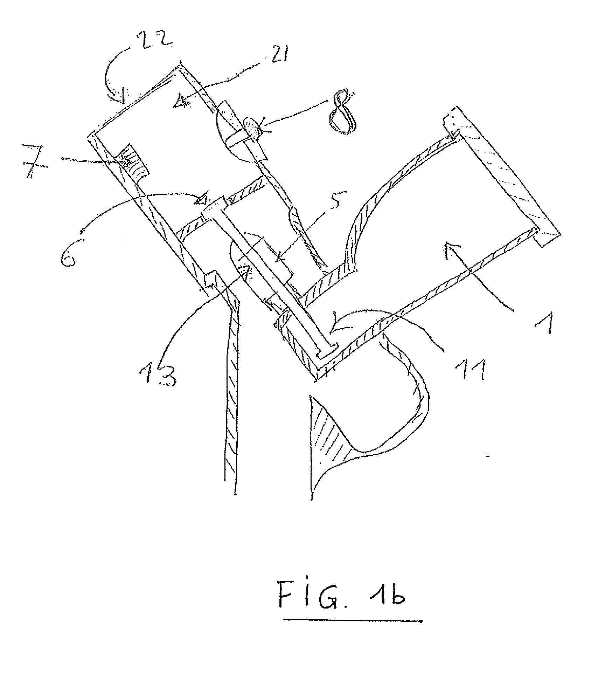

The lower end 3 of the shaft is situated inside said liquid storage vessel 1 in order to be able to withdraw by suction some liquid drug from the vessel 1 in order to minimize the amount of liquid that can not be pumped in the vessel 1. In other words, the lower end 3 of the pump 13 is positioned in the deepest part of the liquid suction chamber 11 of the vessel 1 as shown on Fig. 1a and Fig.1b.

As already mentioned and well known in the prior art, the upper end 2 of the pump shaft 13 is arranged in close vicinity to a mesh member 6, preferably a plate or similar as shown on Fig. 3, having a rear surface 6b and a front surface 6a and comprising at least one, but usually and preferably several, traversing holes 14 extending through said mesh 6 or sieve element, between said rear and front surfaces 6b, 6a so that the liquid can pass trough said holes 14 from the rear surface 6b towards the front surface 6a. Using a mesh member 6 and a vibrating shaft 13 for creating a nebulization jet is also well-known in the art and will not further detailed here.

The mesh plate 6 comprises usually several traversing holes 14 that can be arranged in arrays, in circles, by groups, by spot etc... Further, the mesh member 6 is preferably a disk, but it could have other shapes, such as square, rectangle, ellipse... The position, number, shape... of the multitude of holes 14 of the mesh member 6 are parameters that can be easily chosen by the man of skilled in the art, for example by empirical tests based upon the medical specifications to be reached. Further, the mesh member 6 can be a metal plate, such as stainless steel, nickel-palladium..., or made of a plastic material.

A fixing element can be arranged in the nebulizer, which fixing element applies a mechanical pressure on the front surface 6a of the mesh member 6 thereby maintaining the mesh member in an almost fixed position, i.e. in contact with the upper end 2 of the pump shaft 13. Preferably, the mechanical pressure is applied on the periphery of the mesh member 6 as taught by the document US-A-4,796,807.

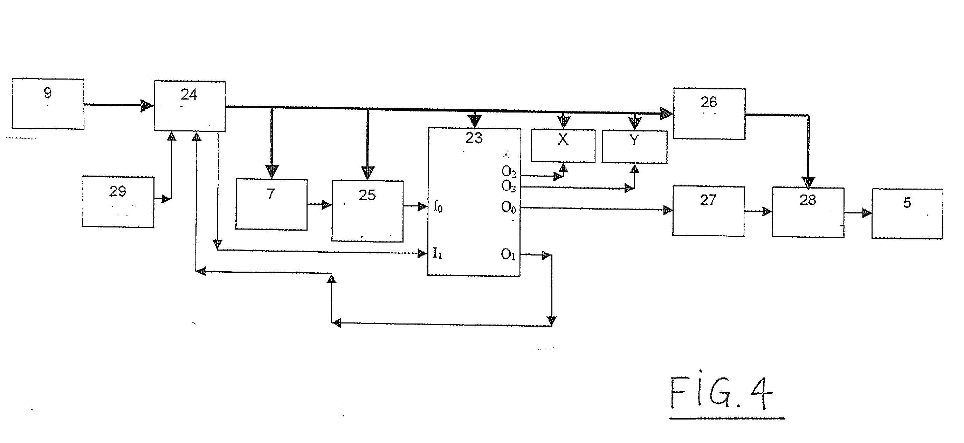

Furthermore, the nebulizer of the present invention also comprises an electronic unit 10. In the preferred but not exclusive embodiment as illustrated on Fig.1a, the electronic unit 10 is arranged in the main body of the nebulizer 20 and comprises, as shown on Fig. 4, a microprocessor 23, which controls the overall operation of the device, a power-supply manager unit 24, an interface circuit with comparator 25, a DC/DC converter 26, a gate 27, an oscillator and vibrator driver 28 and an operation switch 29, and cooperates with sensor 7.

The role of each of the different components of the electronic unit 10 is detailed hereafter.

The electronic unit 10 is powered by a battery unit 9, such as disposable or refillable batteries or similar, arranged in the main body of the nebulizer 20 as shown on Fig. 1a, which main body comprises a housing, for instance made of polymer or similar, comprising the battery unit 9 and the electronic unit 10, and having a shape and dimensions that are chosen such that it can be hold in the user's hand.

According to the present invention, the nebulizer comprises a sensor 7 (or several) for detecting the inspiration phases of the user and more particularly, the start of each inspiration phase of the user. Actually, the detection of the inspiration phases of the user is based on a 'monitoring' of the breathing airflow of the user, as explained below.

The sensor 7 can be incorporated into the main body 20 as shown on Fig. 1a or inside the proximal portion 21 as shown on Fig. 1b and detailed below. Said sensor 7 cooperates, directly or indirectly, with the vibrator 5 for vibrating the pump shaft 13 in response to the detection of such an inspiration phase. In that way, liquid is atomized only when the user is breathing which avoid losses of liquid drug.

The battery unit 9 delivers electrical power to the power-supply manager unit 24 triggered by the press-button switch 29 and latched by the microprocessor 23.

The nebulizer of the invention can actually work in two different modes, i.e. the Continuous Mode (CM) wherein no sensor is used and the Breath-Actuated Mode (BAM) using the sensor 7.

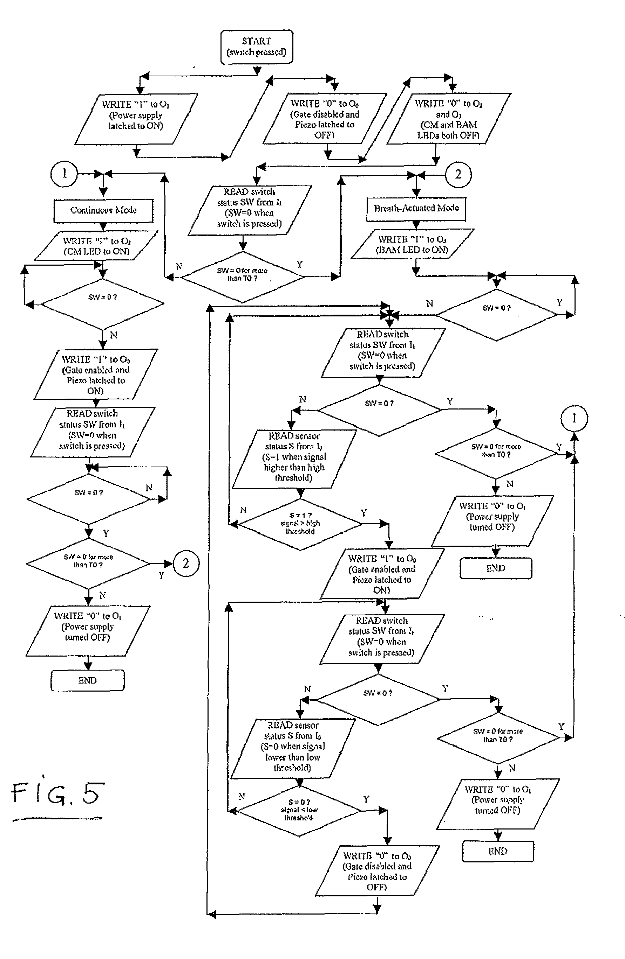

The block diagram and the flow chart of a first embodiment of the nebulizer of invention are shown in Fig. 4 and Fig. 5, respectively, and allow a better understanding of how does the nebulizer work in CM and in BAM modes.

The bold lines of the block diagram correspond to power supply connections, whereas the thin lines correspond to signal connections. Further, the rounded-rectangular blocks of the flow chart indicate "START" and "STOP" ; circular blocks indicate connections referencing to specific points in the diagram ; rectangular blocks indicate processes or actions ; parallelogram blocks correspond to "INPUT" (Read) or "OUTPUT" (Write) operations ; and diamond blocks correspond to decision/test operations.

The selection between "CM" and "BAM" operation modes is performed, at power ON, depending on how long the press-button switch 29 is pressed by the user. Pressing said press-button switch 29 for a duration longer than, i.e. exceeding, a given threshold-time T0, for instance 3 sec, leads to a BAM mode operation, while pressing said press-button switch 29 for less than that the given time T0 leads to a CM mode operation.

The pressing time duration (T) is estimated by the microprocessor 23 through an internal timer and an input I₁ connected to a dedicated output of the power-supply management unit 24.The inputs and the outputs of the microprocessor 23 are digital, i.e. they operate on 1/0 signals.

The dedicated output of the power-supply management unit 24 is a digital signal denoted as the switch status SW, with SW equal to digital 0 and 1, when the press-button switch 29 is pressed and released, respectively.

If the switch status SW is equal to digital 0 for a duration shorter than T0 according to the internal timer, then T<T0 and the microprocessor switches on the outputs O₀ and O₂. As a result, the device is operated in CM operation mode and a CM-mode indicating LED (X) is switched on.

In contrast, if the switch status SW is equal to digital 0 for a duration longer than T0 according to the internal timer, then T>T0 and the microprocessor 23 switches the output O₃ on, at the release of the press-button switch 29. As a result, the device is operated in BAM operation mode and a BAM-mode indicating LED (Y) is switch on.

The two LEDs (X, Y) are arranged on the nebulizer and indicate to the user in which of the two modes the device works, i.e. CM or BAM modes. Of course, in lieu of LEDs, other kinds of indicators can be used, for instance a LCD-display or similar.

In CM mode, the nebulizer works in a classical way, i.e. nebulization occurs all the time until the device is stopped. This operation mode will not be further detailed.

In the BAM mode, the microprocessor 23 becomes ready to be triggered by the interface circuit with hysteresis comparator through the input I₀. Said interface circuit with hysteresis comparator is triggered by the sensor 7 which is a suitable device capable of providing an analog signal proportional, or related monotonically, to the instantaneous breath intensity as actually it is the breath intensity that is used for determining the start and the end of an inspiration phase. Indeed, sensor 7 is capable of providing an analog signal proportional, or related monotonically, to the instantaneous user's airflow.

Said interface circuit with hysteresis comparator 25 internally splits a given threshold level TL into a high threshold H, e.g. 0,3 l/s, and a lower threshold L, e.g. 0,1 l/s, respectively for rising-edge and falling-edge transitions, in order to avoid chattering and ensure clean switching. Fig. 7 gives an example of a typical airflow curve on which the H and L thresholds have been illustrated.

The output of the interface circuit with hysteresis comparator 25 is a digital signal denoted as the sensors status S.

When the signal sent by sensor 7 corresponds to the high threshold H, the interface circuit plus hysteresis comparator 25 sets the sensor status S to digital 1, the microprocessor switches on the ouput O₀ and an enable signal is sent directly to gate 27.

In response to said signal, gate 27 switches on the oscillator and vibrator driver 28 powered by the DC/DC converter 26. Said driver 28 outputs a high frequency (HF) signal of suitable amplitude to the vibrator 5, and the ultrasonic pump device 13, 2, 3, 4, 5 operates to perform the atomization of the liquid through the mesh membrane 6.

Then, when the lower threshold L is reached by the signal from sensor 7, interface circuit with hysteresis comparator 25 resets the sensor status S to digital 0, the microprocessor switches off the output O₀, and gate 27 is disabled and the atomization process is stopped. No atomization or nebulization takes place during the expiration phase as during that phase, i.e. gas are expired and not inspired, which means that the signal from sensor 7 stays well below the L and H thresholds.

The atomization will only start, when the patient starts a new inspiration phase as the breath intensity will successively exceed the H threshold, which will be detected by interface circuit with hysteresis comparator 25 as above explained.

In short, in BAM mode, the detection of the inspiration phases of the user is based on a monitoring of the instantaneous breath intensity by the sensor 7.

Further, with the device of the invention, it is also possible to toggle between the BAM Mode and the CM Mode, and vice versa, by activating mode-toggling means, such as pressing a press-button switch 29 for longer than T0 or another preset duration. However, pressing the press-button switch 29 for shorter than T0 when starting from the "ON" state turns the device "OFF".

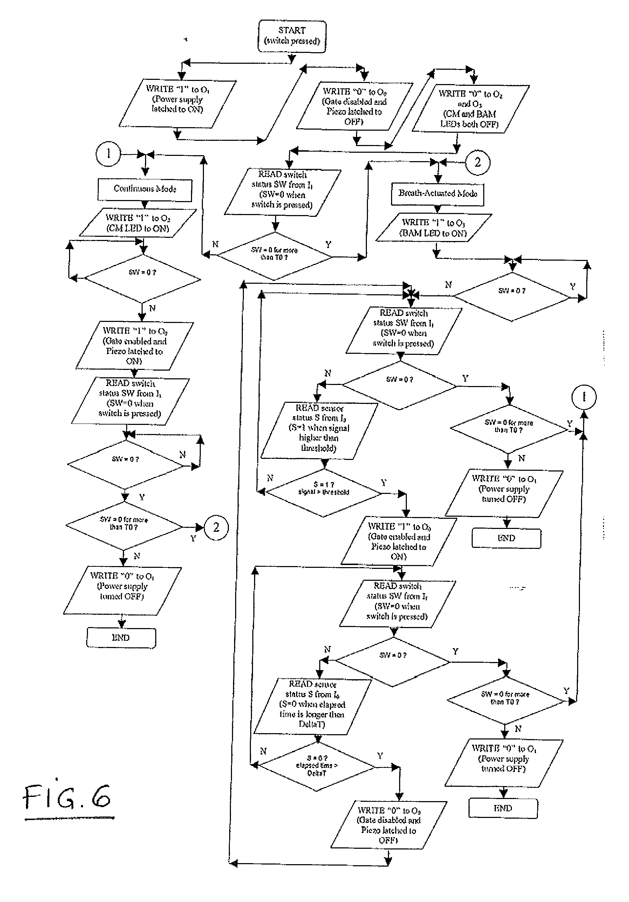

Furthermore, the block diagram and the flow chart of a second embodiment of the invention are shown respectively in Fig. 4 and Fig. 6, wherein the nebulizer can work in the same way and in both CM and BAM modes in the first embodiment, except that, said second embodiment, the interface circuit with hysteresis comparator 25 has been replaced by an interface circuit plus temporized comparator 25 that can trigger the microprocessor 23 through the input I₀.

Said interface circuit plus temporized comparator 25 includes a comparator and a monostable temporized stage and is triggered by the sensor 7 which provides, as in the first embodiment, an analog signal proportional, or related monotonically, to the instantaneous breath intensity.

In other words, in the BAM Mode, the interface circuit with temporized comparator includes a comparator and a monostable temporized stage which activates the nebulizer for a given time duration DeltaT, when an over-threshold event of breath intensity occurs.

The output of interface circuit with temporized comparator is a digital signal denoted as the sensors status S.

When the signal from the sensor 7 exceeds a given threshold level TL, the comparator triggers the interface circuit with temporized comparator, which sets the sensor status S to digital 1 for a given time duration DeltaT.

TL level can be identical to or different from either H or L levels used in the first embodiment.

At the beginning of said time duration DeltaT, the microprocessor 23 switches on the ouput O₀ and an enable signal is sent directly to gate 27, which switches on the oscillator and vibrator driver 28 powered by the DC/DC converter 26. Said driver 28 outputs a high frequency (HF) signal of suitable amplitude to the vibrator 5, and the ultrasonic pump device 13, 2, 3, 4, 5 operates to perform the atomization of the liquid through the mesh membrane 6.

At the end of said time duration DeltaT, the interface comparator monostable block resets the sensor status S to digital 0, and the microprocessor switches off the output O₀, and, as a consequence, gate 27 is disabled and the atomization process is stopped.

Here again, the atomization will only start, when the patient starts a new inspiration phase as explained above.

In other words, in said second embodiment, the duration of the atomization depends on a given prefixed time, i.e. DeltaT, whereas in the first embodiment, it depends on the actual duration of the inspiration phase and more precisely, on when the breathing flow reaches the lower threshold level L.

It should be emphasized that, in both embodiments, the nebulizer can include one or several additional features, such as the threshold level (TL) and DeltaT setting means allowing the user to select or to adjust the threshold level (TL), for instance by either setting a multiple switch or adjusting a suitable trimmer, in order to add flexibility to the device and take into account either the particular breathing pattern of a given user or the particular features or properties of a given drug or class of drugs to be nebulized, or both.

In the nebulizer of Fig. 1a that can work according to the first or second embodiments, the sensor 7 is preferably incorporated into the electronic circuit 10, in a compartment of the main body 20 comprising the electronic unit 10 and it is pneumatically linked to the proximal portion 21 of the device for sensing preferably the gas pressure, i.e. air, in said proximal portion 21. This can be done by means of a thin hollow tube 15 that transmits the measurement to the sensor 7. In such a case, the hollow tube 15 convoys the gas pressure from the proximal portion 21 forming a chamber to the sensor 7, where the pressure variations are detected and can be transformed into electric signal and used as above explained.

However, as shown on Fig.1b, the sensor(s) 7 can also be directly arranged inside the proximal portion 21 and electrically linked (not shown), e.g. by means of electrical connections, like electric wires or cables, to the electronic unit 10 that is arranged in the main body 20 as shown on Fig. 1a.

More generally speaking, a sensor is a device which measures a physical quantity and converts it into a signal which can be read by an instrument. In the spirit of the present application, a complete "measurement" of the relevant parameter related to the breath action is not necessary or mandatory. Indeed, only a simple "detection" of the level or magnitude of said given parameter, such as gas pressure, is needed in order to compare it to one or several given thresholds. This is a less stringent constraint for the system design.

To below listed sensors are possible but not exclusive examples of sensors that can be used in the frame of the present invention for detecting the onset of the inspiration phase of the user.

As the sensor 7, one can use an air flow sensors applied to a conduit properly positioned in front of the patient nose and/or mouth. For instance, a miniaturized air flow sensor based on the thermo-anemometric principle can be used. Such a sensor includes a small central heater, made by a resistor heated by Joule effect, placed between two lateral temperature sensors oriented along the expected air flow. In still air, both temperature sensors are at the same temperature and their differential signal is zero. When air flow is present, the thermal distribution is not anymore symmetrical between the two temperature sensors, with the up-wind sensor going now to a lower temperature because heat is carried away from it by the air flow. In this way, very low flow rates can be accurately detected providing information on both magnitude and direction.

Further, pressure sensors applied to a closed volume put in contact with the patient nose/mouth can also be used. When inspiration takes place, a negative pressure is generated in the volume that is sensed by the pressure sensor. Pressure sensors come in wealth of manufacturing technologies, size and performances. For the present application, piezoresistive pressure sensors could, for example, be used. They are based on stress-sensitive resistors, i.e. piezoresitors or strain-gauges, mounted on a deformable diaphragm that is bended by the pressure to be measured. Piezoresistive pressure sensors respond to variable pressures down to zero frequency, i.e. static or DC pressure. Some of them are manufactured in silicon micromachning technology which can offer a particularly advantageous performance/cost ratio. Some models incorporate extra elements to linearize the response and compensate for temperature effects. As an alternative, capacitive pressure sensors could be used, in which the flexure of the diaphragm caused by the pressure is measured by means of the variation of the sensor capacitance. Capacitive sensors also offer response down to static pressure. Compared to piezoresistive pressure sensors, capacitive sensors may have a lower power consumption. As a further alternative, piezoelectric pressure sensor could be evaluated for use in the present application. They are based on detecting the pressure-induced stress by means of a piezoelectric element. Piezoelectric pressure sensors are typically known as dynamic, or AC, measurement devices, in the sense that they do not respond to pressure variations below a certain low-frequency limit, i.e. they are not sensitive to DC pressure and only respond to pressure variations. This feature can be attractive in the present application where the inspiration act intrinsically generates a pressure variation, irrespective of the static pressure value, but special attention should be paid to electronic signal conditioning in order to accurately detect the stop of inspiration.

Furthermore, acoustic/vibration sensors placed in a conduit properly positioned in front of the patient nose/mouth can be used for detecting the acoustic noise in the conduit caused by the inspiration act. They can be based on piezoelectric elements or pick-ups. Special attention should be paid in considering the effect of environment noise/vibrations that, if not properly shielded, can inadvertently trigger the aerosol device. Acoustic/vibration pick-ups can be cheaper than air flow sensors and pressure sensors, but they may be more problematic to use with reference to the above-mentioned false triggering issue.

All these kinds of sensors 7 are available on the market.

The shape and size of the proximal portion 21 are chosen for allowing an efficient delivery of the atomized liquid to the nostrils of the user, i.e. the patient. As the device should be portable, it must be very light, i.e. having a total weight of less than 0,50 kg, preferably less than 0,25 kg.

Further, as shown on Fig. 1a and Fig. 1b, the proximal portion 21 forming a chamber comprises a one-way exhalation valve 8 for releasing the user's exhalation gas flow, when the user exhales into the chamber of said proximal portion.

Fig. 7 represents the way the nebulizer of the invention works during a breathing cycle of the user, i.e. during the successive inspiratory and expiratory phases, in BAM mode using a nebulizer equipped with an electronic unit 10 as per the first embodiment, i.e. based on H and L treshold detection. Of course, the H value is always greater than the L value.

During an inspiratory phase (insp.), the airflow or breathing intensity sensed by the sensor 7 increases over the time (t) and when it exceeds a given high threshold-value H, for instance of about 0,3 l/sec, the vibrating element is activated (vibrator ON) and the liquid is aerosolized.

At the end of the inspiration phase, the airflow decreases and passes below a given lower threshold-value L, for instance of about 0.1 l/sec. At this time, the vibrating element is stopped (vibrator OFF) and the liquid is no more aerosolized.

During the complete expiration phase (exp.), the airflow is negative and the vibrating element is not vibrated until the start of a new inspiration phase, when the breathing intensity pattern exceeds again the given high threshold-value H.

The nebulizer of the present invention can be set so that the dose of liquid solutions to be aerosolized will be repeatedly released at the same starting "point", e.g. corresponding to an inspiratory user's flow rate of approximately 0,3 l/s for instance (H level), and will stop either after a fixed time, e.g. 2 s (DeltaT), or at a same end point, e.g. corresponding to an inspiratory user's flow rate of approximately 0,1 l/s for instance (L level), with respect to the inspiratory user's flow rate.

The nebulizer of the invention is designed so that the dose of liquid solutions will be aerosolized only when the patient inhales, with an adequate sensitivity to quickly respond to the inhalation. If a patient exhales or coughs, the aerosolization will stop and only resume when the patient begins inhaling again.

An electronic control allows a customization the nebulizer for both relaxed and controlled breathing, facilitating delivery of drug to the desired portion of the lung and reducing the amount of drug losses, thereby allowing for a dose reduction.

The advantages of the breath actuated nebulizer of the invention, especially when working in the BAM mode, are the following :
- virtually no aerosol is produced during user's expiration, which provides a clinical dose assurance and an environmental protection for caregivers and health care providers; and
- less medication is wasted, allowing for a dose reduction.

According to an alternative embodiment (not illustrated), which is less preferred, the nebulizer useable in the frame of the present invention may one of the type comprising a mesh member that is directly vibrated by the vibrating element, such as one or several piezoelectric means, i.e. without need of any pump shaft.

In other words, in said embodiment, the nebulizer comprises a vibrating element cooperating with a mesh member comprising several traversing holes for nebulizing the liquid solution.

As in the previous embodiments, the nebulizer of the alternative embodiment further comprises one (or several) sensor for detecting at least an inspiration phase of the user as explained above. Actually, the detection and the monitoring of the breathing airflow as well as the command of the nebulizer are done as in the previous embodiments by means of the same components, i.e. the electronic circuit 10 and its constituents (See Fig. 4-7).

This alternative embodiment will not be further detailed but it should be emphasized that it can incorporate all the features of the previous embodiments, except that the vibrating element vibrates directly the mesh element in response to the detection of an inspiration phase by said sensor, said mesh being put into contact with the liquid solution to be aerosolized (but without need of any pump shaft), thereby generating the aerosol.

## Claims

1. Nebulizer for delivering a nebulized liquid solution comprising a pump shaft (13) and further comprising a vibrating element (5) for vibrating the pump shaft (13), and a mesh member (6) comprising one or several traversing holes (14) and cooperating with the pump shaft (13) for nebulizing the liquid, **characterized in that** it further comprises at least one sensor (7) for detecting at least an inspiration phase of the user, and the vibrating element (5) vibrates the pump shaft (13) in response to the detection of an inspiration phase by said sensor (7).

2. Nebulizer according to claim 1, **characterized in that** it further comprises a main body (20) having a proximal portion (21) forming a chamber comprising an outlet (22) situated at an extremity of said proximal portion (21), and a vessel (1) for receiving a liquid solution to be nebulized, and pump device (13, 2, 3, 4, 5) comprising the pump shaft (13), and **in that** :
- the pump shaft (13) comprises an upper end (2), a lower end (3) and a pump bore (4) axially arranged through said pump shaft (13) between the upper end (2) and the lower end (3), said lower end (3) being situated inside said vessel (1),
- the mesh member (6) has a rear surface (6b) and a front surface (6a), said rear surface (6b) facing the upper end (2) of the pump shaft (13), and
- the pump shaft (13) being actuated by the vibrating element (5) in such a way that the liquid solution pumped by the pump device (13, 2, 3, 4, 5) in the vessel (1) passes successively through the pump bore (4) and through at least one hole (14) from the rear surface (6b) in the direction of the front surface (6a) of the mesh member (6), and is subsequently atomized in the proximal portion (21) and delivered by the outlet (22) situated at an extremity of said proximal portion (21).

3. Nebulizer according to any one of claims 1 or 2, **characterized in that** it further comprises an electronic unit (10) comprising a microprocessor (23) and an interface circuit with comparator (25) cooperating with sensor (7) for determining the start and/or the end of inspiration and/or expiration phases, preferably the determination of the start and/or the end of an inspiration phase is based on a monitoring of the breath intensity of the user.

4. Nebulizer according to any one of the preceding claims, **characterized in that** the electronic unit (10) further comprises an oscillator and vibrator driver (28) adapted for controlling the vibrating element (5), especially for starting or for stopping a vibration of said vibrating element (5).

5. Nebulizer according to any one of the preceding claims, **characterized in that** the oscillator and vibrator driver (28) is controlled by the microprocessor (23), preferably via a gate (27).

6. Nebulizer according to any one of the preceding claims, **characterized in that** sensor (7) cooperates with an interface circuit with comparator (25) for determining the start of an inspiration phase of the user, the end of an inspiration phase of the user, the start of an expiration phase of the user or the end of an expiration phase of the user, based on a monitoring of the breathing intensity of the user.

7. Nebulizer according to any one of the preceding claims, **characterized in that** the interface circuit with comparator (25) cooperates with microprocessor (23) for controlling the oscillator and vibrator driver (28) so as to start the vibration of the vibrating element (5) in response to the reception by the interface circuit with comparator (25) of a signal coming from sensor (7) indicating that the breathing intensity exceeds a given threshold (H ; TL), preferably said threshold (H ; TL) corresponds to the start of an inspiration phase of the user.

8. Nebulizer according to any one of the preceding claims, **characterized in that** the interface circuit with comparator (25) is an interface circuit with hysteresis comparator, said interface circuit with hysteresis comparator cooperates with microprocessor (23) for controlling the oscillator and vibrator driver (28) so as to stop the vibration of the vibrating element (5) in response to the reception by the interface circuit with hysteresis comparator of a signal coming from sensor (7) indicating that the breathing intensity is below a given low threshold (L), preferably said low threshold (H) corresponds to the end of an inspiration phase of the user.

9. Nebulizer according to any one of claims 1 to 7, **characterized in that** the interface circuit with comparator (25) is an interface circuit with temporized comparator, said interface circuit with temporized comparator cooperates with microprocessor (23) for controlling the oscillator and vibrator driver (28) so as to stop the vibration of the vibrating element (5) after a preset duration (DeltaT).

10. Nebulizer according to any one of the preceding claims, **characterized in that** sensor (7) provides to the interface circuit with comparator (25), an analog signal proportional or related monotonically to the instantaneous breath intensity of the user.

11. Nebulizer according to any one of the preceding claims, **characterized in that** it further comprises a power-supply management unit (24) cooperating with the microprocessor (23) and with a switch (29) that can be actuated by the user, so that the power-supply management unit (24) is able to detect an actuation of the switch (29) by the user and to send a signal to the microprocessor (23) in response to such an actuation.

12. Nebulizer according to any one of the preceding claims, **characterized in that** the microprocessor (23), upon receiving of a signal from the power-supply management unit (24), is able to determine the duration of the actuation of the operation switch (29) by the user and, when the microprocessor (23) determines that the duration of the actuation of the switch (29) by the user exceeds a given value (T0), the nebulizer is operated in breath-actuated mode (BAM) and the sensor (7) starts to detect the inspiration phases of the user.

13. Nebulizer according to any one of the preceding claims, **characterized in that** it further comprises battery means (9) for electrically powering the power-supply management unit (24), the interface circuit with comparator (25), the microprocessor (23), one or several LEDs (X, Y), the DC/DC converter (26) and/or the oscillator and vibrator driver (28).

14. Nebulizer according to any one of the preceding claims, **characterized in that** the sensor (7) is pneumatically linked to the proximal portion (21) for sensing the gas pressure in said proximal portion (21), preferably by means of a hollow tube (15) that transmits at least measurement to the sensor (7), or **in that** said sensor (7) is situated inside said proximal portion (21) and electronically linked to said electronic unit (10).

15. Nebulizer according to any one of the claims 1 to 13, **characterized in that** it comprises mode-toggling means for toggling between the BAM mode and the CM mode, and vice versa, preferably the mode-toggling means comprise a press-button switch (29).

16. Process for delivering a nebulized liquid solution by means of a nebulizer comprising a pump shaft (13), a vibrating element (5) for vibrating the pump shaft (13), a vessel (1) for receiving a liquid solution to be nebulized, and a mesh member (6) comprising traversing holes (14) and cooperating with the pump shaft (13) for nebulizing the liquid, comprising the steps of:
a) using at least one sensor (7) for detecting at least an inspiration phase of the user,
b) vibrating the pump shaft (13) by means of the vibrating element (5) in response to the detection of an inspiration phase by said sensor (7) thereby nebulizing at least part of the liquid solution.

17. Nebulizer for delivering a nebulized liquid solution comprising a vibrating element cooperating with a mesh member comprising several traversing holes for nebulizing the liquid solution, **characterized in that** it further comprises at least one sensor for detecting at least an inspiration phase of the user, and the vibrating element vibrates the mesh in response to the detection of an inspiration phase by said sensor.
